# EUROPÄISCHE PATENTSCHRIFT

(11) **EP 0 863 157 B1**
(45) Veröffentlichungstag und Bekanntmachung des Hinweises auf die Patenterteilung: **07.04.2004**
(21) Anmeldenummer: 97122273.2
(22) Anmeldetag: 17.12.1997
(51) Int. Cl.: C07K 16/28, C12N 5/20, G01N 33/577, G01N 33/574, A61K 39/395, A61P 35/02

(54) **Anti-Megakaryozyten monoklonaler Antikörper 97A6**
Anti-megakaryocyte monoclonal antibody 97A6
Anticorps monoclonal 97A6 contre des mégakaryocytes

(30) Priorität: 05.03.1997 DE 19708877
(43) Veröffentlichungstag der Anmeldung: 09.09.1998
(73) Patentinhaber: Eberhard-Karls-Universität Tübingen Universitätsklinikum, 72076 Tübingen (DE)
(72) Erfinder: Bühring, Hans-Jörg, Dr., 72076 Tübingen (DE)
(74) Vertreter: Otten, Hajo, Dr.-Ing.

(56) Entgegenhaltungen:
- VAN DEN OUDENRIJN S. ET AL.: "Reactivity of the blind monoclonal antibody panel of the platelet section with megakaryocytic cell lines and cultured CD34+ cells" TISSUE ANTIGENS - 6TH INTERNATIONAL WORKSHOP AND CONFERENCE ON HUMAN LEUKOCYTE DIFFERENTIATION ANTIGENS, Bd. 48, Nr. 4-2, 10. - 14.November 1996, KOBE, JAPAN , Seite 470 XP002065240
- WINDERBANK K. ET AL.: "Acute megakaryocytic leukemia (M7) in children" MAYO CLINICAL PROCEEDINGS, Bd. 64, Nr. 11, 1989, Seiten 1339-1351, XP002065241
- CONTI R. CLINICAL CARDIOLOGY Bd. 22, 1999, Seiten 57 - 58

## Beschreibung

Die Erfindung betrifft einen monoklonalen, gegen die Megakaryozyten-Zellinie UT-7 gerichteten Antikörper.

Ähnliche Antikörper sind inzwischen allgemein bekannt, sie sind gegen Megakaryozyten-spezifische Antigene gerichtet.

Megakaryozyten sind außergewöhnlich große Zellen mit einem Durchmesser von bis zu 60 µm, die sich im menschlichen Knochenmark befinden.

In einem als Hämatopoese oder Blutbildung bezeichneten Prozeß gehen Megakaryozyten, ebenso wie Lymphozyten, Monozyten und Erythrozyten, aus pluripotenten Stammzellen hervor. Megakaryozyten gehören der myeloischen Reihe der Hämatopoese an. Sie differenzieren sich aus einer für alle myeloischen Zellen gemeinsamen Vorläuferzelle, der sogenannten CFU-GEMM (colony forming unit: granulocytes, erythrocytes, monocytes, megakaryocytes). Zur Bildung der verschiedenen ausdifferenzierten Effektorzellen durchlaufen die myeloischen Stammzellen jeweils spezifische Differenzierungsprogramme.

Die Hämatopoese aller Zellen der myeloischen und lymphatischen Reihe findet im Knochenmark statt. Bis auf die Megakaryozyten wandern jedoch alle ausdifferenzierten myeloischen Zellen aus dem Knochenmark ins Blut ab.

Die im Knochenmark verbleibenden Megakaryozyten entwickeln Ausläufer, die direkt in die Blutgefäße des Knochenmarks hineinragen. Von diesen Ausläufern werden die Thrombozyten, auch Blutplättchen genannt, abgegeben und von dort aus im gesamten Blutsystem verteilt. Die Thrombozyten stellen somit die letzte Differenzierungsstufe der Megakaryozyten dar. Sie sind jedoch keine Zellen im eigentlichen Sinne, sondern lediglich Zellfragmente. Die physiologische Aufgabe der Thrombozyten ist das Starten der Blutgerinnungskaskade.

Die Unterscheidung der verschiedenen Zelltypen in Knochenmark und Blut sowie deren Zuordnung in verschiedene Differenzierungsstadien spielt im Klinikalltag eine wesentliche Rolle. So ist es bspw. zur Diagnose von Blutbildungsstörungen notwendig, die Zellzahl jedes spezifischen Zelltyps und möglichst auch das jeweilige Differenzierungsstadium zu erfassen.

Darüber hinaus ist die Untersuchung der Blut- und Blutvorläuferzellen im Knochenmark bei der Diagnose von Leukämien wichtig, Anzahl, Art und Stadium der Zellen werden zur Klassifizierung oder Zuordnung des Leukämietyps sowie zur Entscheidung über eine angemessene Therapie herangezogen. Dabei richtet sich die Zuordnung von Leukämien einerseits nach dem klinischen Verlauf der Krankheit, und andererseits nach dem Reifegrad und der Abstammung der pathologisch veränderten Leukozyten. Dazu ist es jedoch notwendig, Zelltyp und Status sowohl der gesunden als auch der entarteten, in einer Patientenprobe enthaltenen Zellen zu erfassen.

Bisher erfolgen diese Analysen entweder im Mikroskop anhand der Morphologie der Zellen, nach Anfärbung mit klassischen Färbemethoden, bspw. der Pappenheim-Färbung, und durch manuelles Auszählen. Modernere Verfahren zur Auswertung von Knochenmarksbiopsien oder Blutproben verwenden Antikörper, die spezifische Antigene als Marker für bestimmte Zelltypen und -stadien erkennen. Die Antikörper können dann in Standardverfahren wie dem ELISA (Enyzme linked immunosorbent assay) oder der Durchflußzytometrie (FACS-Analyse, fluorescence activated cell sorting) automatisiert nachgewiesen werden.

Die wesentlichste Voraussetzung zur Anwendung derartiger Verfahren ist es, geeignete Antikörper zur Hand zu haben, die eine hohe Sensitivität und vor allem Spezifität für die verschiedenen Zelltypen und -stadien aufweisen und die darüber hinaus in großen Mengen zur Verfügung stehen.

Ein Problem bei der Analyse von Knochenmarks- und Blutzellen stellen die Megakaryozyten und vor allem ihre "Ableger", die Thrombozyten, dar. Es sind verschiedene Antikörper bekannt, die gegen Megakaryozyten-spezifische Antigene gerichtet sind, wie eingangs bereits erwähnt wurde. Diese Antikörper erkennen teilweise zusätzlich auch Thrombozyten, so daß mit diesen Antikörpern eine Unterscheidung von Megakaryozyten und Thrombozyten nicht möglich ist.

Ein weiteres Problem ergibt sich dadurch, daß sich Thrombozyten auch an andere Blutzellen oder Vorläuferzellen, vor allem an Monozyten, anlagern. So werden Monozyten fälschlich als Megakaryozyten oder Thrombozyten identifiziert, und sowohl die Monozyten- als auch die Megakaryozytenanalyse liefert falsche Ergebnisse.

Van den Oudenrijn et al., "Reactivity of the blind monoclonal antibody panel of the platelet section with megakaryocytic cell lines and cultured CD34+ cells", 1996, 6^{th} International Workshop and Conference on Human Leukocyte Differentiation Antigens, Conference Book 48, 470, erwähnt einen mit p50 bezeichneten monoklonalen Antikörper, der an Megakaryozyten-Zellinien bindet, jedoch nicht Thrombozyten erkennt. Dem Fachmann wird in dieser Veröffentlichung jedoch keinerlei Hinweis darauf gegeben, wie zu einem solchen Antikörper gelangt werden kann, noch wurde ein entsprechender Antikörper zum Zeitpunkt dieser Veröffentlichung z.B. durch ordnungsgemäße Hinterlegung offenbart und damit der Öffentlichkeit zugänglich gemacht.

Vor diesem Hintergrund ist es Aufgabe der vorliegenden Erfindung, einen Antikörper bereitzustellen, der ein Antigen auf der Megakaryozyten-Zellinie UT-7 erkennt, und der in praktisch unbegrenzter Menge zur Verfügung steht.

Diese Aufgabe wird durch die Bereitstellung eines monoklonalen Antikörpers gelöst, der von Hybridomzellen produziert und freigesetzt wird, die unter der Nr. DSM ACC2297 am 12.02.1997 bei der Deutschen Sammlung von Mikroorganismen und Zellkulturen GmbH (DSMZ) hinterlegt sind. Er ist mit der Bezeichnung 97A6 benannt.

Mit dem erfindungsgemäßen Antikörper wurde erstmals ein derartiger monoklonaler Antikörper bereitgestellt, der standardisiert reproduzierbar ist und somit potentiell unbegrenzt hergestellt werden kann.

Darüber hinaus bietet der erfindungsgemäße Antikörper nun die Möglichkeit, die Diagnose von Blutbildungsstörungen und Tumoren, insbesondere Leukämien, zu erleichtern.

Die Erfindung betrifft ferner Hybridomzellen, die einen erfindungsgemäßen monoklonalen Antikörper erzeugen. Sie umfaßt insbesondere die Hybridomzellen, die unter der Nr. DSM ACC2297 bei der Deutschen Sammlung von Mikroorganismen und Zellkulturen GmbH (DSMZ) hinterlegt sind und den Antikörper mit der Bezeichnung 97A6 produzieren.

Hybridomzellen, die einen erfindungsgemäßen Antikörper synthetisieren und freisetzen, werden durch ein Verfahren hergestellt, das die im Stand der Technik grundsätzlich geläufigen Schritte umfaßt, wie sie bspw. von Bührung et al. in Hybridoma 1991, Band 10, Nr. 1, S. 77-78 beschrieben wurden:
a) Immunisierung oder Sensibilisierung eines Tieres, vorzugsweise einer Maus vom Balb/c-Stamm, mit dem Antigen bzw. Immunogen;
b) Gewinnung der antikörperproduzierenden Zellen, vorzugsweise der Milzlymphozyten dieses Tieres;
c) Fusionierung dieser antikörperproduzierenden Zellen mit einer stabilen, immortalisierten Zellinie, vorzugsweise einer Myelomzellinie, zu Hybridomzellen; und
d) Vereinzelung und Vermehrung (Klonierung) solcher Hybridomzellen, die einen Antikörper sezernieren, der an das Antigen bindet.

Dabei wird das Tier mit Zellen der erythro-/megakaryoblastischen Zellinie UT-7 immunisiert.

Die Erfindung betrifft auch die Verwendung eines spezifischen gegen Megakaryozyten gerichteten Antikörpers zur Analyse der Hämatopoese in Patientenproben.

Mit Hilfe des erfindungsgemäßen Antikörpers ist es nunmehr möglich, einen spezifischen Weg der Hämatopoese zu untersuchen. Die Verwendung des erfindungsgemäßen Antikörpers ist nicht nur für den Forscher interessant, der die Differenzierungsprozesse untersucht. Diese Anwendung des erfindungsgemäßen Antikörpers spielt auch im klinischen Diagnoselabor eine Rolle, wenn anhand von zellulärem Material eines an einer Blutbildungsstörung leidenden Patienten festgestellt werden muß, welcher Zelltyp bzw. welcher Schritt der Hämatopoese bei ihm gestört ist.

In einer bevorzugten Ausgestaltung wird der erfindungsgemäße Antikörper dazu verwendet, die zelluläre Zusammensetzung von Patientenproben, insbesondere von Knochenmarksbiopsien und/oder Blutproben zu analysieren.

Dem in vielen Kliniken und Laboratorien eingesetzten Routineverfahren zur Bestimmung der zellulären Zusammensetzung in Patientenproben mit Hilfe von ELISA- oder FACS-Geräten wird somit ein Antikörper mit bisher noch nicht erreichter Spezifität zur Verfügung gestellt.

In einer bevorzugten Ausführung wird der Antikörper zur diagnostischen Einordnung von Leukämien in Patientenproben verwendet.

Die Diagnose und Klassifizierung von Leukämien erfolgt anhand von Knochenmarksbiopsien oder Blutproben. So wird bspw. bei einer Ausprägungsform der Leukämie, der chronisch-myeloischen Leukämie (Abkürzung CML), anhand von Knochenmarksuntersuchungen der Status aller myeloischen Zellen, also der Granulozyten, Monozyten, Erythrozyten und Megakaryozyten, analysiert.

Um die diagnostische Applikation des erfindungsgemäßen Antikörpers zu erleichtern, kann der Antikörper mit entsprechend geeigneten Hilfssubstanzen in einer pharmazeutischen Zubereitung vermischt sein. Die Erfindung betrifft deshalb auch ein diagnostisches Mittel, das einen erfindungsgemäßen Antikörper enthält. Vorzugsweise enthält dieses pharmazeutische Mittel einen Antikörper, wie er von den unter der Nr. DSMZ ACC2297 bei der Deutschen Sammlung von Mikroorganismen und Zellkulturen GmbH, DMSZ, hinterlegten Hybridomzellen produziert und freigesetzt wird.

In einer besonders bevorzugten Ausführung des diagnostischen Mittels ist der erfindungsgemäße Antikörper mit einem Marker, insbesondere mit einem Fluoreszenzmarker, verbunden.

Hierbei ist vorteilhaft, daß der Antikörper dann mit hoher Sensitivität nachgewiesen werden kann, weshalb nur kleine Mengen des diagnostischen Mittels zur Diagnose eingesetzt werden müssen. Außerdem ist bei Verwendung eines solchen diagnostischen Mittels die Diagnose mit Hilfe von automatisierten ELISA-Geräten und Durchflußzytometern möglich.

Darüber hinaus betrifft die Erfindung auch ein Verfahren zur Analyse der zellulären Zusammensetzung einer Patientenprobe, mit den Schritten:
a) Inkubation der Patientenprobe mit einem diagnostischen Mittel, in dem ein mit einem Marker verbundener, erfindungsgemäßer Antikörper enthalten ist; und
b) Nachweis des Markers, vorzugsweise durch ELISA- oder FACS-Analyse.

Dieses Verfahren hat den Vorteil, daß damit die Bestimmung von Zellen in Patientenproben hochsezifisch, sensitiv und schnell erfolgt und in automatisierter Form durchführbar ist.

Weitere Vorteile ergeben sich aus der nachstehenden Beschreibung.

Die Erfindung wird im folgenden anhand von Anwendungs- und Ausführungsbeispielen unter Heranziehung der Figuren näher erläutert. Es zeigen:
- Fig. 1: sieben Histogramme aus durchflußzytometrische Untersuchungen über die Expression von siebe verschiedenen Zelloberflächenstrukturen der Zel linie UT-7; und
- Fig. 2: Histogramme der durchflußzytometrischen Untersu chungen der gleichen sieben Oberflächenstrukture wie in Fig. 1, die jedoch mit einer Sublinie vo UT-7 mit hoher Expression des 97A6 Antigens er halten wurden.

### Beispiel 1: Herstellung und Charakterisierung erfindungsgemäßer monoklonaler Antikörper

Als Antigen werden Zellen der erythro-/megakaryoblastischen Zellinie UT-7 verwendet (Komatsu N. et al. Establishment of a human leukemic cell line with megakaryocytic features: Dependency on granulocyte-macrophage colony-stimulating factor, interleukin-3, or erythropoetin for growth and survival, Cancer Res. 1991; 51: 341-348).

Acht Wochen alte Balb/c-Mäuse werden zweimal in Intervallen von zehn Tagen intraperintoneal mit 10⁷ Zellen der Zellinie UT-7 immunisiert. Vier Tage vor der Fusion werden 5 x 10⁵ Zellen direkt in die Milz appliziert, um die Immunantwort zu verstärken.

Die Antikörper-Bildung im Mausorganismus wird dadurch überprüft, daß das Blutserum des betreffenden Tieres in dem dem Fachmann geläufigen ELISA-Test auf Bindungseigenschaften mit dem Antigen untersucht wird.

Nach ca. drei Wochen werden die Lymphozyten des erfolgreich immunisierten Tieres gewonnen, indem die Milz herausoperiert und zu einer Zellsuspension zerkleinert wird.

Die suspendierten Milzzellen werden in Anwesenheit von Polyethylenglykol mit Myelomzellen des bekannten Stammes SP2/0 fusioniert. Die Fusionskultur wird in Hypoxanthin-, Aminopterinund Thymidin-(HAT-)haltigem Medium, hier in HAT-RPMI-1640, kultiviert, in dem sich nur hybride Zellen vermehren können, da diese sowohl die Eigenschaften der Myelomzellen zur unbegrenzten Teilungsfähigkeit als auch die Eigenschaft der antikörperproduzierenden Lymphozyten zum Wachstum in HAT-haltigem Medium haben.

Nach der Fusion werden die Zellen in Mikrotiterplatten ausplattiert und bei 37 C und 5 % CO₂ inkubiert.

Die Kulturüberstände werden nach 10 bis 14 Tagen auf der Zellinie UT-7 im Durchflußzytometer gescreent. Hybridome, die Antikörper mit der gewünschten Spezifität produzieren, werden ausgewählt und nach dem bekannten Grenzverdünnungsverfahren vereinzelt und kultiviert, d.h. kloniert.

Positiv reagierende Hybridomzellkulturen werden weiter kultiviert, die Antikörper angereichert, gereinigt und charakterisiert.

Nach der obigen Screening-Strategie wurde der monoklonale Antikörper 97A6 erhalten. Der Isotyp wurde über PE-konjugierte Anti-Isotyp-spezifische Antiseren durch direkte Immunfluoreszenz zu IgGl bestimmt.

Die Produktion, Reinigung und Charakterisierung der Antikörper erfolgte mit den in Fachkreisen allgemein bekannten Methoden. Der Antikörper 97A6, der von den unter der Nr. DSM ACC2297 bei der Deutschen Sammlung von Mikroorganismen und Zellkulturen GmbH, DSMZ, hinterlegten Hybridomzellen erzeugt wird, weist die folgenden charakteristischen Merkmale auf:

| | |
|---|---|
| Immunglobulinklasse | IgG1 |
| Bindungsaffinität an | Antigen der Megakaryozyten-Zellinie UT-7 |

### Beispiel 2: Screening von etablierten Zellinien auf Erkennung durch den Antikörper 97A6

Um die Spezifität des Antikörpers 97A6 zu untersuchen, wurden insgesamt 48 verschiedene etablierte Zellinien auf ihre Reaktivität mit dem Antikörper 97A6 hin analysiert. Die Ergebnisse sind unten in Tabelle 1 aufgelistet.

Die Abstammung sowie die entscheidenen Eigenschaften jeder einzelnen Zellinie waren bekannt, so daß dieses Experiment nicht nur die Spezifität des Antikörpers beleuchtet, sondern auch Aufschluß über die Expression des 97A6-Antigens gibt.

Die Reaktion der Zellen mit dem Antikörper 97A6 wurde im Durchflußzytometer (FACS-Gerät) gemessen.

**Tabelle 1:**

| Reaktion des Antikörpers 97A6 mit verschiedenen Zellinien | | | |
|---|---|---|---|
| Zellinie | Art der Zellinie | Eigenschaft der Zellinie | Erkennung durch 97A6 |
| DU.4475 | Tumorzellinie | Brustkarzinom (epithelial) | - |
| T-47D | Tumorzellinie | Brustkarzinom | - |
| MCF-7 | Tumorzellinie | Brustkarzinon | - |
| NCI-H128 | Tumorzellinie | SCLC (Small Cell Lungenkrebs) | - |
| NCI-H69 | Tumorzellinie | SCLC (Small Cell Lungenkrebs) | - |
| SK-MES | Tumorzellinie | Lungenkarzinom (squamous) | - |
| Calu 1 | Tumorzellinie | Lungenkarzinom (epidermoid) | - |
| Calu 3 | Tumorzellinie | Lungenkarzinom (adeno) | - |
| Calu 6 | Tumorzellinie | Lungenkarzinom (adeno) | - |
| SK-LU1 | Tumorzellinie | Lungenkarzinom (adeno) | - |
| HELA | Tumorzellinie | Cervixkarzinom (epithelial) | - |
| 5637 | Tumorzellinie | Blasenkarzinom (epithelialähnlich) | - |
| IMR-32 | Tumorzellinie | Neuroblastom (Fibroblastenund Neuroblastenähnlich | - |
| TE-671 | Tumorzellinie | Medulloblastom (epithelialähnlich | - |
| A172 | Tumorzellinie | Glioblastom | - |
| U138 | Tumorzellinie | Glioblastom | - |
| A431 | Tumorzellinie | Glioblastom | - |
| **MKPL1** | leukämische Zellinie: erythroblastisch/megaka ryozytisch | megakaryoblastische Leukämie | + |
| **KU.812** | leukämische Zellinie: erythroblastisch/megaka ryozytisch | megakaryozytische/basophile Leukämie Ph+* (CML)** | + |
| **UT-7** | leukämische Zellinie: erythroblastisch/megaka ryozytisch | erythro-/megakaryozytische Leukämie | + |
| TF-1 | leukämische Zellinie: erythroblastisch/megakaryozytisch | erythro-/megakaryozytische Leukämie | - |
| M07e | leukämische Zellinie: erythroblastisch/megakaryozytisch | erythro-/megakaryozytische Leukämie | - |
| **MEG-01** | leukämische Zellinie: erythroblastisch/megakaryozytisch | erythro-/megakaryozytische Leukämie Ph+* (CML)** | + |
| MOLM-1 | leukämische Zellinie: erythroblakaryozytisch | erythro-/megakaryozytische Leukämie Ph+* (CML)** | - |
| K562 | leukämische Zellinie: erythroblastisch/megakaryozytisch | erythro-/megakaryozytische Leukämie Ph+* (CML)** | - |
| HEL | leukämische Zellinie: erythroblastisch/megakaryozytisch | erythro-/megakaryozytische Leukämie Ph+* (CML)** | - |
| EM2 | leukämische Zellinie: myeloblastisch | myeloblastisch, Ph+* (CML)** | - |
| KG-1 | leukämische Zellinie: myeloblastisch | myeloblastische Leukämie | - |
| KG-1a | leukämische Zellinie: myeloblastisch | unreife Sublinie von KG-1 | - |
| HL60 | leukämische Zellinie: myeloblastisch | myeloblastische Leukämie | - |
| DU.528 | leukämische Zellinie: myeloblastisch | myeloblastische Leukämie | - |
| U937 | leukämische Zellinie: myeloblastisch | myeloblastische Leukämie | - |
| OCI/AML-4 | leukämische Zellinie: myeloblastisch | myeloblastische Leukämie | - |
| CML-T1 | leukämische Zellinie: T-lymphoblastisch | T-lymphoblastisch, Ph+* (CML)** | - |
| HSB-2 | leukämische Zellinie: T-lymphoblastisch | T-lymphoblastische Leukämie | - |
| CCRF-CEM | leukämische Zellinie: T-lymphoblastisch | T-lymphoblastische Leukämie | - |
| Molt-4 | leukämische Zellinie: T-lymphoblastisch | T-lymphoblastische Leukämie | - |
| Jurkat | leukämische Zellinie: T-lymphoblastisch | T-lymphoblastische Leukämie | - |
| Daudi | leukämische Zellinie: B-lymphoblastisch | EBV-transformierte B-Zelllinie | - |
| Reh | leukämische Zellinie: B-lymphoblastisch | pre-B-lymphoblastische Leukämie | - |
| 207 | leukämische Zellinie: B-lymphoblastisch | pre-B-lymphoblastische Leukämie | - |
| 380 | leukämische Zellinie: B-lymphoblastisch | pre-B-lymphoblastische Leukämie | - |
| 697 | leukämische Zellinie: B-lymphoblastisch | pre-B-lymphoblastische Leukämie | - |
| Km3 | leukämische Zellinie: B-lymphoblastisch | pre-B-lymphoblastische Leukämie | - |
| BV-173 | leukämische Zellinie: B-lymphoblastisch | pro-B-lymphoblastisch, Ph+* (CML)**, (CD10+) | - |
| Nalm-1 | leukämische Zellinie: B-lymphoblastisch | pre-B-lymphoblastisch, Ph+* (CML)**, (CD10+) | - |
| U266 | leukämische Zellinie: B-lymphoblastisch | Myelomzellinie | - |

| | | | |
|---|---|---|---|
| * Ph+ bedeutet Philadelphia Chromosom-positiv | | | |
| ** CML bedeutet chronisch-myeloische Leukämie | | | |

Die Ergebnisse dieser intensiven Untersuchungen zeigen, daß der Antikörper 97A6 hochspezifisch mit vier Zellinien mit megakaryozytischen Eigenschaften reagiert, nämlich den Linien UT-7, K0.812, MKPL1 und MEG-01.

### Beispiel 3: Analyse der Bindung verschiedener monoklonaler Antikörper an megakaryozytische Zellinien

Insgesamt 14 verschiedene monoklonale Antikörper wurden mit Hilfe der Durchflußzytometrie auf ihre Reaktivität mit verschiedenen megakaryozytischen Zellinien hin analysiert. Einer der getesteten Antikörper war der Antikörper 97A6, in dieser Studie P50 genannt. Dieser Antikörper wurde von dem Erfinder zur Verfügung gestellt.

Die eingesetzten etablierten Zellinien repräsentieren nach derzeitigem Wissensstand verschiedene Differenzierungsstadien der Megakaryozytenentstehung von undifferenzierten Stammzellen (CD34⁺-Zellen, Zellinie KG1a) bis hin zu den Thrombozyten. Diese Untersuchungen sind der Veröffentlichung von Sonja van den Oudenrijn et al.: Reactivity of the blind monoclonal antibody panel of the platelet section with megakaryocytic cell lines and cultured CD34⁺ cells In: Leukocyte Typing VI, Kishimoto T. et al., eds. Garland Publishing, Inc. New York (1997), in press, entnommen. Die Ergebnisse sind in Tabelle 2 aufgeführt.

**Tabelle 2**

| Reaktion verschiedener Antikörper mit megakaryozytischen Zellinien | | | | | | | |
|---|---|---|---|---|---|---|---|
| eingesetzte Antikörper | getestete Zellinien | | | | | | |
| | KG1a | K562 | HEL | CHRF | MKPL | MEGO | Thrombozyten |
| CD41 (6C9) | 32 | - | 16 | 74 | 90 | 51 | 99 |
| CD42b (MB45) | - | 13 | - | 10 | 11 | - | 98 |
| CD63 (435) | 98 | 94 | 78 | 36 | 94 | 79 | 14 |
| P48(11B2.G4) | 100 | 94 | 93 | 94 | 96 | 85 | 95 |
| P49(14A2.H1) | 96 | 80 | 30 | 25 | 81 | 53 | 86 |
| P50(**97A6**) | - | - | - | - | 81 | 66 | - |
| P51(Ad2/ 13H12) | 12 | - | - | - | - | 22 | 19 |
| P52(ALMA.7) | - | - | - | - | - | - | 91 |
| P53(HIP10) | 35 | 42 | 39 | 76 | 78 | 56 | 94 |
| P54(HIP11) | 27 | - | 15 | 68 | 74 | 50 | 97 |
| P55(NaM12-6B6) | - | - | - | - | - | - | 94 |
| P56(NaM28-8C12) | - | - | 18 | - | 87 | 16 | 94 |
| P57(NaM81-1D10) | - | - | - | - | - | - | 23 |
| P58(UM.8D2) | 98 | 88 | 96 | 99 | 87 | 63 | 62 |

Die Reaktivität der Antikörper wird als Prozentsatz an positiven Zellen angegeben, wobei ein Minuszeichen eine nicht signifikante Bindung von weniger als 10 % repräsentiert.

Diese Studie zeigt, daß der Antikörper 97A6, in Tabelle 2 P50 genannt, der einzige Antikörper ist, der mit megakaryozytischen Zellinien, nicht jedoch mit Thrombozyten reagiert.

### Beispiel 4: Herstellung einer Sublinie von UT-7 mit hoher Expression des 97A6-Antigens

Da bei der Herstellung der Hybridomzellinie, die den monoklonalen Antikörper 97A6 herstellt, ganze Zellen der Zellinie UT-7 zur Immunisierung verwendet wurden, ist das entsprechende Antigen offenbar ein membranständiges Zelloberflächenprotein dieser Zellinie.

Das Antigen wird außerdem auf drei weiteren Zellinien exprimiert, darunter die Zellinie MKPL1 (siehe Beispiel 1).

Es wurde eine stärker exprimierende Zellinie hergestellt, aus der eine Expressionsbibliothek erstellt werden kann, um daraus dann das entsprechende für das Antigen kodierende Gen zu identifizieren.

Dazu wurden UT-7-Zellen mit dem Antikörper 97A6 und einem fluoreszenzmarkierten zweiten Antikörper inkubiert und danach 5 % der Zellen mit der höchsten Antigendichte mit Hilfe eines FACSvantage-Zellsortierers herausselektioniert.

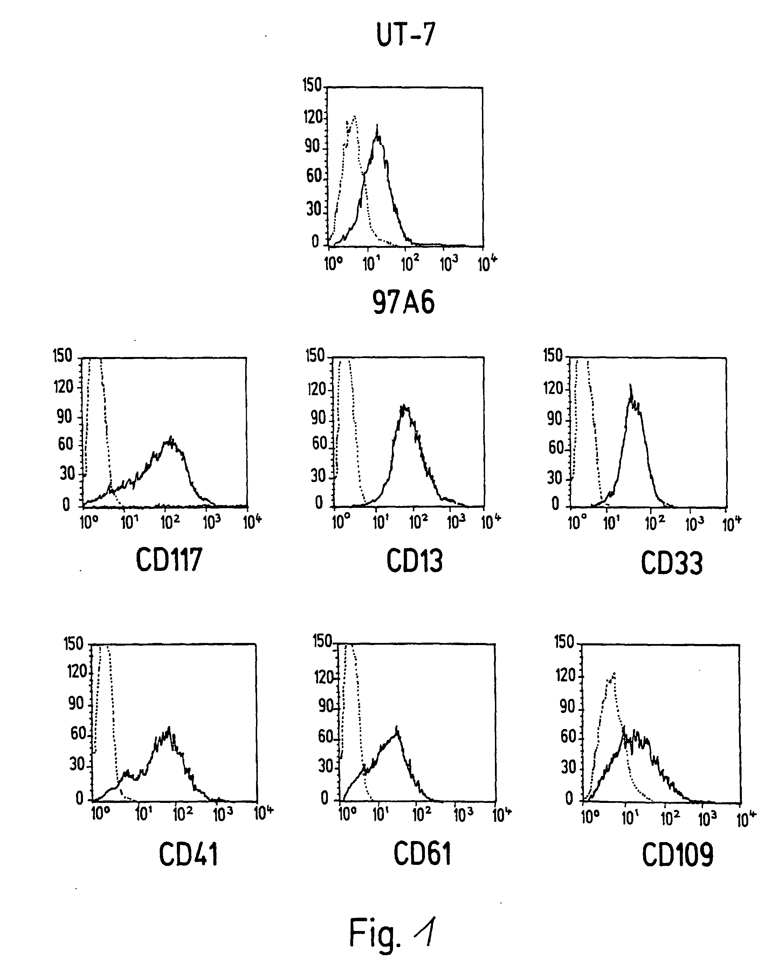

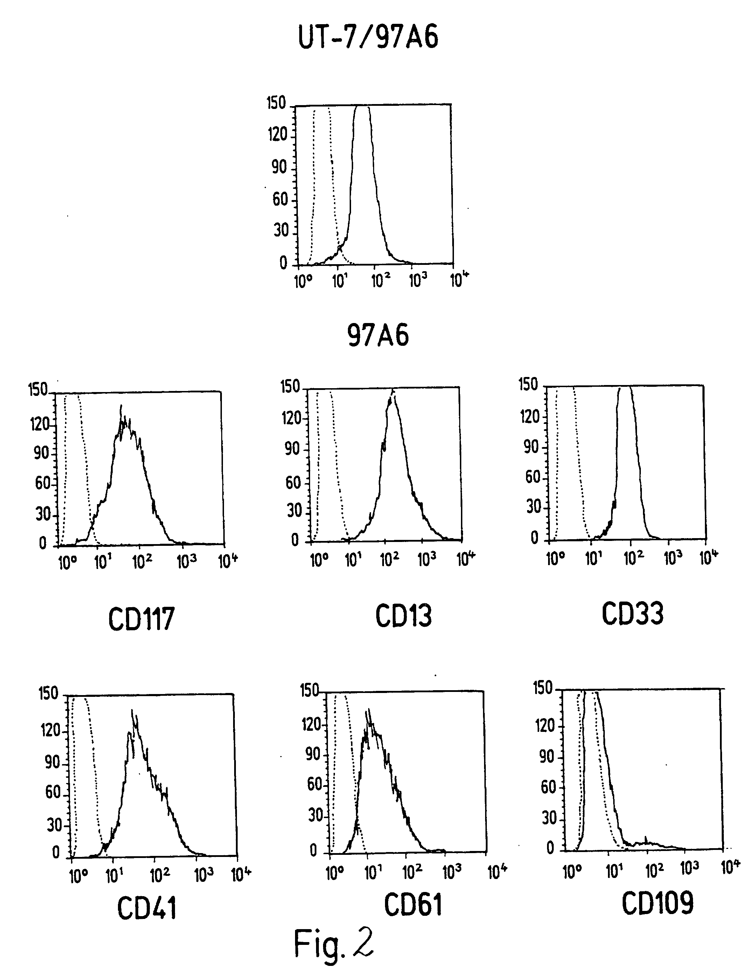

Die Zellen wurden dann in Kultur vermehrt. Nach Erhalt von 10⁷ Zellen wurde die Prozedur wiederholt. Die daraus hervorgegangene Zellinie wurde UT-7/97A6 genannt. Die Ergebnisse der Analyse der Zellinien UT-7 und UT-7/97A6 im Durchflußzytometer sind in den Fig. 1 und 2 gezeigt. Darin ist das Ausmaß der Expression des 97A6-spezifischen Antigens sowie die Expression von sechs weiteren Antigenen in der Ausgangszellinie UT-7 (Fig. 1) sowie in der ausselektionierten Zellinie UT-7/97A6 (Fig. 2) gezeigt. Die sich aus den Histogrammen dieser FACS-Analysen ergebenden Fluoreszenzintensitäten, die die Expressionsraten der Antigene widerspiegeln, sind in Tabelle 3 zusammengefaßt.

**Tabelle 3:**

| Expression verschiedener Antigene auf UT-7 und UT-7/97A6 | | | |
|---|---|---|---|
| Analysiertes Antigen | Art des Markers | mittlere Fluoreszenzintensität auf UT-7 | mittlere Fluoreszenzintensität auf UT-7/97A6 |
| 97A6-spezifisches Antigen | | 26,44 | 67,14 |
| CD117 | Stammzellmarker | 128,9 | 77,02 |
| CD109 | Stammzellmarker | 29,3 | 9,36 |
| CD13 | myeloischer Marker | 144,61 | 291,73 |
| CD33 | myeloischer Marker | 42,28 | 103,35 |
| CD41 | megakaryozytischer Marker | 70,61 | 100,54 |
| CD61 | megakaryozytischer Marker | 25,8 | 33,9 |

Diese Ergebnisse zeigen, dass die mit Hilfe des Antikörpers 97A6 isolierte Sublinie der Zellinie UT-7 das 97A6-Antigens mit einer ungefähr dreimal so hohen Dichte wie die Ursprungszellinie exprimiert. Die erzeugte Zellinie UT-7/97A6 weist außerdem Eigenschaften auf, die für eine in der Differenzierung fortgeschrittene megakaryozytische Zelle typisch sind. Die Stammzellmarker CD117 und CD109 sind in der Subzellinie wesentlich geringer exprimiert als in der Ursprungszellinie, während die myeloischen Marker CD13 und CD33 sowie die megakaryozytischen Marker CD41 und CD61 stärker auf der erzeugten Sublinie exprimiert sind. Daraus ergibt sich, dass UT-7/97A6 eine reifere Zellinie als die Ursprungszelllinie darstellt.

Diese Zellinie dient nun als Ausgangsmaterial zur Herstellung einer Expressionsbibliothek, aus der die codierenden Sequenzen des Antigens isoliert werden können.

Die Nukleinsäure- bzw. Aminosäuresequenz gibt dann Aufschluss über die Natur und möglicherweise auch die Funktion des 97A6-Antigens.

## Patentansprüche

1. Monoklonaler Antikörper, der von Hybridomzellen produziert und freigesetzt wird, die am 12.02.1997 unter der Nr. DSM ACC2297 bei der Deutschen Sammlung von Mikroorganismen und Zellkulturen GmbH, DSMZ, gemäß dem Budapester Vertrag hinterlegt worden sind.

2. Hybridomzellen, **dadurch gekennzeichnet, daß** sie die Fähigkeit aufweisen, einen Antikörper gemäß Anspruch 1 zu erzeugen.

3. Hybridomzellen nach Anspruch 2, **dadurch gekennzeichnet, daß** sie am 12.02.1997 unter der Nr. DSM ACC2297 bei der Deutschen Sammlung von Mikroorganismen und Zellkulturen GmbH, DSMZ, gemäß dem Budapester Vertrag hinterlegt sind.

4. Verwendung eines Antikörpers nach Anspruch 1 zur Analyse der Hämatopoese in Patientenproben.

5. Verwendung eines Antikörpers nach Anspruch 1 zur Analyse der zellulären Zusammensetzung von Patientenproben, insbesondere von Knochenmarksbiopsien und/oder Blutproben.

6. Verwendung eines Antikörpers nach Anspruch 1 zur diagnostischen Einordnung von Leukämien in Patientenproben.

7. Diagnostisches Mittel zur Verwendung nach einem der Ansprüche 4 bis 6, **dadurch gekennzeichnet, daß** es einen Antikörper gemäß Anspruch 1 enthält.

8. Diagnostisches Mittel nach Anspruch 7, **dadurch gekennzeichnet, daß** der Antikörper mit einem Marker, insbesondere mit einem Fluoreszenzmarker, verbunden ist.

9. Verfahren zur Analyse der zellulären Zusammensetzung von Patientenproben, **gekennzeichnet durch** die Schritte
a) Inkubation der Proben mit einem diagnostischen Mittel nach Anspruch 8 und
b) Nachweis des Markers **durch** ELISA- oder FACS-Analyse.

## Claims

1. A monoclonal antibody, being produced and released by hybridoma cells deposited on February 12, 1997 at the Deutschen Sammlung von Mikroorganismen und Zellkulturen GmbH, DSMZ, under No. DSM ACC2297, in accordance with the Budapest Treaty.

2. Hybridoma cells, **characterized in that** they have the ability to produce an antibody according to claim 1.

3. Hybridoma cells according to claim 2, **characterized in that** they are deposited on February 12, 1997 at the Deutschen Sammlung von Mikroorganismen und Zellkulturen GmbH, DSMZ under No. DSM ACC2297, in accordance with the Budapest Treaty.

4. Use of an antibody according to claim 1 for analysis of the hematopoesis in patients' samples.

5. Use of an antibody according to claim 1 for analysis of the cellular composition of patients' samples, especially of bone marrow biopsies and/or blood samples.

6. Use of an antibody according to claim 1 for diagnostic classification of leukemias in patients' samples.

7. A diagnostic means for usage according to any of claims 4 to 6, **characterized in that** it contains an antibody according to claim 1.

8. Diagnostic means according to claim 7, **characterized in that** the antibody is connected to a marker, especially to a fluorescent marker.

9. A method for analyzing the cellular composition of patients' samples, **characterized by** the steps of
a) incubation of the samples with a diagnostic means according to claim 8, and
b) detection of the marker via ELISA or FACS analysis.

## Revendications

1. Anticorps monoclonal produit par des cellules d'hybridome et libéré de celles-ci, qui ont été déposées le 12.02.1997 sous le numéro DSM ACC2297 auprès de la Deutsche Sammlung von Mikroorganismen und Zellkulturen GmbH, DSMZ, conformément au traité de Budapest.

2. Cellules d'hybridome, **caractérisées en ce qu'**elles présentent la capacité de produire un anticorps selon la revendication 1.

3. Cellules d'hybridome selon la revendication 2, **caractérisées en ce qu'**elles ont été déposées le 12.02.1997 sous le numéro DSM ACC2297 auprès de la Deutsche Sammlung von Mikroorganismen und Zellkulturen GmbH, DSMZ, conformément au traité de Budapest.

4. Utilisation d'un anticorps selon la revendication 1, pour l'analyse de l'hématopoïèse dans des échantillons provenant de patients.

5. Utilisation d'un anticorps selon la revendication 1 destinée à l'analyse de la composition cellulaire d'échantillons provenant de patients, en particulier de biopsies de moelle osseuse et/ou de prélèvements sanguins.

6. Utilisation d'un anticorps selon la revendication 1 pour le classement diagnostique des leucémies dans des échantillons provenant d'un patient.

7. Moyen de diagnostic destiné à l'utilisation selon l'une quelconque des revendications 4 à 6, **caractérisé en ce qu'**il contient un anticorps selon la revendication 1.

8. Moyen de diagnostic selon la revendication 7, **caractérisé en ce que** l'anticorps est lié à un marqueur, en particulier à un marqueur fluorescent.

9. Procédé pour l'analyse de la composition cellulaire d'échantillons provenant de patients, **caractérisé par** les étapes constituées de
a) l'incubation des échantillons avec un moyen de diagnostic selon la revendication 8 et
b) la détection du marqueur par analyse ELISA ou FACS.
